# EUROPEAN PATENT SPECIFICATION

(11) **EP 3 517 138 B1**
(45) Date of publication and mention of the grant of the patent: **27.04.2022**
(21) Application number: 18305067.3
(22) Date of filing: 26.01.2018
(51) Int. Cl.: A61L 2/08, A61L 2/00

(54) **PHOTOBIOMODULATION DEVICE**
PHOTOBIOMODULATIONSVORRICHTUNG
DISPOSITIF DE PHOTOBIOMODULATION

(43) Date of publication of application: 31.07.2019
(73) Proprietor: URGO RECHERCHE INNOVATION ET DEVELOPPEMENT, 21300 Chenôve (FR); Universität Heidelberg, 69117 Heidelberg (DE)
(72) Inventor: BOUSCHBACHER, Marielle, 21220 Chambolle-Musigny (FR); STEINBRUNN, Julien Denis Marie, 21380 Messigny-et-Vantoux (FR); LAVIGNE, Louis, 87000 Limoges (FR); GRETZ, Norbert, 68159 Mannheim (DE); BECKER, Anja, 68165 Mannheim (DE); HULLEN, Andreas, 69123 Heidelberg (DE); KLAPCZYNSKI, Anna, 68167 Mannheim (DE); KUCH, Natalia, 68159 Mannheim (DE); MU, Yifei, 69189 Mannheim (DE)
(74) Representative: Plasseraud IP

(56) References cited:
- WO-A1-2007/012875
- WO-A2-2005/034855
- US-A1- 2014 343 478
- US-A1- 2017 080 117

## Description

### TECHNICAL FIELD

This invention relates to an *in vitro* method comprising exposing a contaminating and/or pathogenic agents to a light source device able to reduce contaminating and/or pathogenic agents' growth and number, notably for the treatment of liquid or fluid medium (such as used waters) or for the decontamination or disinfection of various supports (including inert or biologic surfaces, for instance, wounds, skin or mucosa) preferably through a photobiomodulation mean.

### BACKGROUND OF THE INVENTION

The disclosure relates to the use of visible light, in the wavelength (λ) range of 440 to 490 nanometers (nm), in combination with highly specific fluence and power density to create a specific light, which can reduce the growth and the number of contaminating and/or pathogenic agents on any support or in any medium.

Bacteria, such as *Pseudomonas aeruginosa, Staphylococcus aureus* or *Escherischia coli,* or more generally, contaminating and/or pathogenic agents, are responsible for the development of various human and animal diseases or disturbances of their global Health and well-being. For example, the growth and the proliferation of these bacteria's species can lead to urinary, pulmonary, digestive or skin infections or disorders of the skin or wounds.

Generally, different forms of light can be used in many different applications. Through the delivery of specific wavelengths of light, effects such as the inactivation of microorganisms such as bacteria, yeasts or fungi, viruses and parasites can be accomplished.

Some specific wavelengths of visible light can be used for active reasons beyond general illumination. For example, activation of fluorescent materials can be achieved with approximately 400 to approximately 420 nm range, similar to UVA, or a "black light," curing of plastics can be achieved with approximately 380 to approximately 420 nm light, heat delivery can be achieved with near-infrared approximately 650 to approximately 700 nm light, and inactivation of bacteria can be achieved with near-infrared approximately 650 to approximately 700 nm light. Regarding inactivation of bacteria, millions of hospital patients contract a hospital-acquired infection (HAI) from bacterial, viral, or fungal microorganisms. Environmental contamination in hospital environments is a key factor in the source of these HAIs, among others. Current methods of attacking environmental contamination range widely, from traditional mopping and surface cleaning to the use of burst ultraviolet (UV) and hydrogen peroxide vapor. Yet, in full force application, infections are still a reality in almost every hospital.

In the cultivation of livestock and agricultural products, contamination from bacteria, fungi, or viruses can cause losses of animal life, plant life, and/or spoilage of rendered products. Common production practices now pack animals and plants densely for efficiency, in terms of space and finances, yet contamination from microorganisms can spread rapidly in such an environment, with infection spreading between plants or animals. Currently, there is extensive use of pesticides, antibiotics, and chemical cleaners to prevent loss of final product by preventing contamination of animals or agriculture products, yet animal and plant losses, final product losses, and the unknown distribution of contaminated final products is still an issue faced by the industry. Thus, there is a continuing need for better methods to control microorganisms in the cultivation environment and processing facilities to prevent loss of final product.

In the retail sale of food, fresh products are commonly displayed to customers in the shopping environment. In many retail stores, the products are stored on shelves and in cases with viewing windows. Many of these products are considered perishable, with a very short shelf life like meat, produce, or fish. The short shelf life of these items is due to the degradation of the quality of the product over the time displayed. This degradation of the product is caused by a variety of factors: breakdown of cells or molecules due to aging, loss of water or other volatile components into the air, or spoilage based on bacterial, fungal, or viral contamination.

Controlled environments are required for many purposes, such as the preservation of food products, the aging of goods, such as wines, liquors, and tobacco products, general prevention of contamination during many industrial processes, or in medical treatments such as during wound healing process. Such environments are protected and controlled in many different manners, including in terms of air quality, temperature, humidity, and particle count.

Perishable food is commonly stored in refrigerated enclosures to slow degradation of the food and to slow bacterial growth and proliferation that can cause food spoilage and food sickness. While refrigeration alone can extend food life and quality, compared with room temperature storage, bacteria and molds can still be common destroyers of food in these environments, in a home refrigerator just as in an industrial meat locker.

In the field of cosmetics or pharmaceuticals, products must meet preservation standards in regards of microbial proliferation to ensure sufficient shelf life and microbial cleanliness of the products. One of the methods to meet this preservation standard is to include preservatives in the product, such as parabens or phenoxyethanol.

However, these preservatives can be poorly tolerated and may be considered potentially endocrine disruptors. Therefore, the prevention of the growth and proliferation of bacteria or fungi within cosmetic or dermatologic formulations without using preservatives would be of high value. This is even more important for customer or patient with highly reactive skin or atopic dermatitis who have to use formulations without any preservatives as they can cause skin irritation or even allergy.

Humidors are humidity controlled environments, commonly associated with the storage or aging of cigars and tobacco products, that maintain moisture content at a set level for the items stored in the enclosure. However, bacterial and mold spoilage of these goods can occur in the event of contamination, resulting in the loss of what is typically a high value product.

In clean rooms, efforts are made to control the amount of particles in the air in a given enclosure. Most of them function by continuously pumping in filtered air and forcing the exodus of airborne particles. Bacterial growth and number and the generation of bacteria or mold spores from contaminated sites can continuously generate particles in the environment that can be difficult to prevent and cause costly contamination issues in high-value products undergoing processing or storage in the environment.

In a food preparation environment (e.g., restaurants, industrial kitchens, fast food, prepared goods store, for direct sale or delivery to the consumer/customer) bacteria, fungi, parasites and/or viruses pose issues of spoilage, pathogenic contamination, and infection, and can be a serious issue for the establishment. These contamination issues can come from a large variety of sources in such an open environment: e.g., personnel, customers, raw materials, air systems, and water. While many cleaning practices have typically been implemented at these sites, contamination and infection outbreaks are still seen. Typically, these contamination issues are only noticed after the damage is done, when inventory is spoiled or customers are sick.

Another interesting use of light is linked to the clothing items and the necessity to clean any cloth or shoe from any contaminating agent.

In the medical field, the prevention of growth and proliferation of contaminating and/or pathogenic agents is mostly obtained by the use of topically applied antiseptic agents.

UV light can be used for disinfection in an industrial or medical environment, but its reductive effects on any contaminating and/or pathogenic agents' growth and proliferation could still be further improved.

The effect of light emitting in the UV or violet wavelength has already been disclosed in the patent application WO 2009/056838. This document describes a device that emits in specific wavelength comprised between 380 and 420 nm, preferably at 405 nm, said device comprising LEDs having a power density of 10 mW/cm² and providing an effective fluence of at least 40 J/cm² for reducing the growth and the number of different species of bacteria.

Nevertheless, UV light is known as being a human carcinogen and may cause DNA damages such as mutations.

In addition, a publication made by Guffey and Wilborn (2006) "In Vitro Bactericidal Effects of 405-nm and 470-nm Blue Light", in Photomedicine and Laser Surgery. 24(6): 684-688; discloses the difficulty of providing a desired reduction of growth and number of different species of bacteria using different wavelengths, typically using dominant emission wavelength at 405 and 470 nm (within violet and blue spectral respectively) and using different fluence values. Indeed, this document describes how the number of colonies of different species of bacteria chosen from *S*. *aureus* (Gram-positive bacteria) or *P. aeruginosa* (Gram-negative bacteria) can evolve differently when exposed to different wavelength and different fluence values. More precisely, this document exhibits a drastic reduction of the number of colonies of *P. aeruginosa* or *S*. *aureus* at a wavelength of 405 nm, whatever the fluence. On the contrary, at a wavelength of 470 nm, the number of colonies of *P. aeruginosa* does not decrease with the increase of fluence. These effects are not similarly reproduced against *S*. *aureus.*

It appears therefore difficult to find a standard method that permits to significantly reduce the growth and the number of contaminating and/or pathogenic agents of a treated support or medium, that would provide reproducible results whatever the agent specie (ie providing a reduction of growth and number of agents for example to any Gram-positive or Gram-negative specie). There would therefore be a need for a standard method capable of providing a significant and reproducible reduction of the growth and proliferation of contaminating and/or pathogenic agents, whatever the nature or specie of said agents, said device being safe and harmless towards human health.

### SUMMARY OF THE INVENTION

It was surprisingly discovered that the growth and the number reduction of contaminating and/or pathogenic agents (including microorganisms (such as bacteria, yeasts or fungi), organisms (such as parasites, dust mite, worm, and louse) and viruses) obtained with blue light could be significantly improved by irradiating said contaminating and/or pathogenic agents with a specific wavelength range light and under specific conditions. In particular, contaminating and/or pathogenic agents is preferably a microorganism such as bacteria, yeasts or fungi, preferably bacteria. More preferably, the contaminating and/or pathogenic agent is a bacteria, in particular a Gram-positive or Gram-negative bacteria, preferably chosen from *S*. *aureus* and *P. aeruginosa.* Blue light is generally known for its anti-proliferative effect, but the inventors demonstrated that the anti-contaminating and/or anti-infectious effects could be further significantly improved using a specific dominant emission wavelength, irradiance and fluence, leading to a significant growth and number reduction of contaminating and/or pathogenic agents with applications to the treatment of liquid or fluid medium such as used water, or of supports such as surface decontamination or disinfection of skin, wounds, mucosa, preferably through a photobiomodulation means.

Photodynamic therapy is a method that uses a photosensitizer, or photosensitizing agent, which is disposed or injected near the treated medium or support, more specifically near the skin, mucosa or the wound and activated by a light at a specific wavelength. Photosensitizers have the ability to interact with contaminating and/or pathogenic agents when exposed to a light at a specific wavelength. Photodynamic therapy is thus an indirect phototherapy because the light is provided to the photosensitizer to treat a medium or support containing a contaminating and/or pathogenic agent, but the light is not directly provided to this medium or support.

Photobiomodulation is a method to provide a biological effect on support or in a medium, preferably over the skin, mucosa or the wound, directly, which means without the need of providing any product or composition to transpose or potentialize the biological effect engendered by the light source. This method can be distinguished from the photodynamic therapy which needs absolutely and every time the intervention of an intermediate product (photosensitizer or a photosensitizing agent) between the light source and support or medium to potentialize the biological effect of the light on said support or medium. In other words, in photobiomodulation, light has a direct effect on support or on the contaminating and/or pathogenic agent whereas, in photodynamic therapy, light has an indirect effect on said support or contaminating and/or pathogenic agents via the activated photosensitizer. As mentioned in the technical field above, the present invention is preferably directed to photobiomodulation.

The unexpected technical effect is achieved by employing a light source device comprising a light emitting element emitting a blue light having a wavelength ranging from 440 to 490 nm, the light source device providing an effective fluence to the contaminating and/or pathogenic agents greater than 11 J/cm² and a power density greater than 20 mW/cm².

According to another embodiment, the dominant emission wavelength ranges from 450 to 460 nm.

According to another embodiment, the light source device provides an effective fluence to any contaminating and/or pathogenic agent greater than 40 J/cm², preferably greater than 80 J/cm². According to another embodiment, the light emitting element provides a power density ranging from 20 to 150 mW/cm² and more particularly a power density ranging from 23 to 46 mW/cm², especially for use in any device able to contact the skin, wound or mucosa. According to the invention, the ratio between the effective fluence and the power density of the light emitting element is greater than 1.7 J/ mW preferably greater than 3 J/ mW.

According to another embodiment, said light emitting element comprises at least one LED.

According to another embodiment, the light source device comprises a power source providing electrical power to said light emitting element.

According to another embodiment, said power source may be a battery, a solar cell, or anything that can produce a power source.

According to another embodiment, the light source device comprises at least one among a microchip processor, a control unit, a communication unit, an external port and a sensor.

The light source used in the method of the present invention may be part of a light source assembly comprising a product adapted to be in contact with a support, in particular a surface and said light source device as described above connected to the product to provide light to at least one contaminating and/or pathogenic agent. The product is one among a dressing, a strip, a compression means, a band-aid, a patch, a gel, a film-forming composition and a rigid or flexible support.

According to another embodiment, the invention is directed to the use of said light source device for reducing the contaminating and/or pathogenic agent's growth and number. In particular the invention is directed to the use of said light source device according for the treatment of fluid or liquid medium or surface decontamination, preferably through a photobiomodulation means. More specifically, the invention is directed to the use of said light source device according or said light source assembly, for the disinfection, preferably through a photobiomodulation means.

### BRIEF DESCRIPTION OF THE DRAWINGS

Figure 1 represents in a cross section view an embodiment of a photobiomodulation device used in the treatment of various supports or medium for promoting the growth and number reduction of contaminating and/or pathogenic agents in vitro or in vivo.
Figure 2 represents a histogram comparing the effect on colony count of E. *Coli* in blue light irradiation conditions (453 nm, defined as BLI) Vs without irradiation (defined as control), and for irradiance values of 23 mW/cm².
Figure 3 represents a histogram comparing the effect on colony count of E. *Coli* in blue light irradiation conditions (453 nm, defined as BLI) Vs without irradiation (defined as control), and for irradiance values of 10 mW/cm².

### DETAILED DESCRIPTION

The present invention will be described below relative to several specific embodiments. Those skilled in the art will appreciate that the present invention may be implemented in a number of different applications and embodiments and is not specifically limited in its application to the particular embodiment depicted herein.

For the purpose of the present invention, the following terms are defined.

The term "Wavelength" is the distance between two peaks of a wave. The symbol for wavelength is λ (lambda) and the unit of measurement is nanometers (nm).

The term "Dominant emission wavelength" is the wavelength or a narrow range of wavelengths the light source emits the majority of the time. The term "power" refers to the rate at which work is perform; the unit of power is Watt (W) and since the light output power is low it is expressed in milliwatts (mW).

The term "power density" or "light intensity", or "irradiance", or "exitance" is the power divided by the area of the target being illuminated by the light and is expressed in mW/cm².

The term "fluence" or "energy density" or "dose" expressed in Joules per cm² (J/cm²) is the product of power (mW) and time per spot size (cm²).

The term "transmitted fluence" is the fluence produced by the claimed light source, whereas the term "effective fluence" is the fluence actually received by the contaminating and/or pathogenic agent. Indeed, as will be further explained below, the effective fluence may be lower than the transmitted fluence depending, in particular, on the environment (medium or support) of the agent.

The term "photobiomodulation" is the ability of the light source device to have a biological effect on cells, or on contaminating and/or pathogenic agents, directly, which means without the need of any provisional product or composition to transpose or potentialize any biological effect engendered by the light source. This term can be distinguished from the term of "photodynamic therapy" which needs absolutely and every time the intervention of an intermediate product between the light source and the cells or contaminating and/or pathogenic agent to potentialize the biological effect of the light.

The term «contaminating and/or pathogenic agent» is intended to designate any microorganism such as bacteria, yeast or fungi, any virus or any organisms in contact with a skin, wound or mucosa such as parasite, louse, dust mite or worm.

By "contaminating agent", we intend to qualify any one of the «contaminating and/or pathogenic agent» listed above able to grow and proliferate on a specific support or in a specific medium (including an inert surface, a pharmaceuticals composition, a biological surface, more particularly a skin, a mucosa or a wound).

By "pathogenic agent", we intend to qualify any contaminating agent capable of inducing a disease or a biological trouble to an animal or a human being.

The term "support" is intended to designate any substrate or surface on which a contaminating and/or pathogenic agent can grow and proliferate, including an inert surface, a biological surface more particularly a skin, a mucosa or a wound.

The term "medium" is intended to designate any environment in which bacteria can grow and proliferate, including a pharmaceuticals composition, used waters, or the air.

The term "microorganism" is intended to designate bacteria, yeasts, and fungi.

The expression "growth and number reduction of contaminating and/or pathogenic agent" means that microorganisms, parasites and viruses, preferably bacteria, growth and number can be limited. This ability can be characterized by a bacterial reduction of at least 0.1 log or 20%.

A first object of the invention is an *in vitro* method for inhibiting growth and reducing number of contaminating and/or pathogenic agents, the method comprising the step of exposing the contaminating and/or pathogenic agents to a light source device (10) comprising a light emitting element (12) emitting a blue light having a wavelength ranging from 440 to 490 nm, and
the light source device (10) providing an effective fluence to the contaminating and/or pathogenic agents greater than 11 J/cm², and a power density greater than 20 mW/cm²,
wherein the ratio between the effective fluence and the power density is greater than 1.7 J/ mW preferably greater than 3 J/ mW.

The light source device 10 is able to emit light at wavelengths within the range of 440-490 nm, preferably within a specific dominant emission wavelength of 450-460 nm. More particularly, the chosen dominant emission wavelength may be 450 or 453 nm.

Furthermore, the light source device 10 is used in the method of the invention to provide light to contaminating and/or pathogenic agent present on the support or medium C at irradiance and fluence (dose or energy density) able to at least inhibit this growth and number in said medium or support. The fluence at which light is provided to the contaminating and/or pathogenic agents present on the support or medium C corresponds to the specific conditions, particularly specific conditions of irradiance and exposure with a light source having a specific dominant emission wavelength ; allowing to obtain the unexpected technical effect with regard to the prior art. Indeed, it was observed that monitoring the irradiance of the provided light allows having a growth and number-reductive effect on irradiated contaminating and/or pathogenic agents.

The growth and number reduction of contaminating and/or pathogenic agents may be performed on any support or in any medium, *ex vivo* or *in vivo.* Indeed, contaminating and/or pathogenic agents may be present in liquid medium such as used waters or the air, on any inert surfaces or in human or animal tissues, in particular on wounds.

The light source device 10 used in the method of the invention provides light at a specific fluence and power density to *in vitro* contaminating and/or pathogenic agents such as bacteria to provide the growth and number-reductive effect.

Depending on many interference means, as described above, disposed between the contaminating and/or pathogenic agent and the light source, the effective fluence of the light received by said agent may be lower than the fluence transmitted by the light emitting element. Indeed, it was also observed that a larger fluence has to be generally transmitted by the light emitting element 12 to provide a predetermined fluence of light to the contaminating and/or pathogenic agent on the support or medium C, i.e. an effective fluence of light adsorbed by the contaminating and/or pathogenic agent. Indeed, during the emission, a part of the light is adsorbed by other elements than the contaminating and/or pathogenic agent which induces a loss of light. Therefore, the light source device 10 provides light at a transmitted fluence so that the contaminating and/or pathogenic agent receives a predetermined fluence (also called effective fluence). Depending on the elements that can be present between the light emitting element and the target contaminating and/or pathogenic agent, the attenuation or absorption effect of the light may lead to an attenuation ranging from 20% to 60% or from 30% to 50% of the energy density, preferably around 45%.

To obtain the unexpected growth and number-reductive effect of the contaminating and/or pathogenic agent, the irradiance or power density is of at least 20 mW/cm², particularly in the range from 20 to 150 mW/cm² and more particularly in a range from 23 mW/cm² to 46 mW/cm².

The effective dose or fluence received by the contaminating and/or pathogenic agent, in particular a bacteria of a wound or a given surface of skin tissue, may be of at least 11 J/cm², and preferably from about 40 J/cm² to about 600 J/cm², or about 41 J/cm² to about 590 J/cm², or about 42 J/cm² to about 580 J/cm², or about 45 J/ cm² to about 570 J/ cm², about 50 J/ cm² to about 560 J/cm², or about 55 J/cm² to about 550 J/cm², or about 60 J/ cm² to about 540 J/cm², or about 65 J/cm² to about 530 J/cm², or about 70 J/ cm² to about 520 J/cm², or about 75 J/cm² to about 510 J/cm², or about 80 J/ cm² to about 500 J/cm², or any light dose in a range bounded by, or between, any of these values. Preferably, the effective fluence used to treat target contaminating and/or pathogenic agent, and preferably bacteria is greater than 40 J/cm², preferably greater than 80 J/cm².

As indicated above, the fluence (dose or energy density) notably depends on both irradiance (mW/cm²) and time. Therefore, obtaining the predetermined fluence may be accomplished by using a higher power light source, which may provide the needed energy in a shorter period of time, or a lower power light source may be used for a longer period of time. Thus, a longer exposure to the light may allow a lower power light source to be used, while a higher power light source may allow the treatment to be done in a shorter time.

The duration of radiation or light exposure administered to a medium or support containing the contaminating and/or pathogenic agent, may also vary. In some embodiments, the exposure ranges from at least 1 second, at least few seconds, or at least 30 minutes, or at least 30, 31, 32, 33, 34, 35, 36, 37, 38, 39, 40, 41, 42, 43, 44, 45, 46, 47, 48 minutes; or up to about 5 hours, 4h, 3h, 2h, 1h or, for any amount of time in a range bounded by, or between, any of these values.

According to a specific embodiment, the light source device used in the method of the invention is used in the growth and number reduction of contaminating and/or pathogenic agents under specific conditions. Particularly, it was observed that the growth and number-reductive effect occurs on contaminating and/or pathogenic agents when provided with an effective fluence greater than 11 J/cm² with a power density of about 20 mW/cm² during about 10 minutes to 2h. Preferably, it was observed that the growth and number-reductive effect occurs on contaminating and/or pathogenic agents when provided with an effective fluence greater than 40 J/cm² with a power density from about 23 mW/cm² to about 46 mW/cm² during about 30 minutes to 2h.

According to a specific embodiment of the invention, the light source device used in the method of the invention is able to emit light continuously (for instance one time, providing specific fluence values), sequentially (for example many times, separated by defined latencies, providing specific fluence values) or by means of pulsations (for example one or many times, providing a specific fluence depending on opposite variations of irradiance and time of exposure values).

According to the present invention, the ratio between the effective fluence and the power density of the light source device of the invention is greater than 1.7 preferably greater than 3. The ratio between the effective fluence and the power density of the light source device characterizes the energy regarding the irradiance received by the contaminating and/or pathogenic agent, preferably the bacteria. It is an indicator qualifying the performance of the treatment.

For thermal issues, the light source device used in the method of the present invention may irradiate the contaminating and/or pathogenic agent either continuously or in pulses. Indeed, pulsed light irradiation will typically be preferred than continuous light if there are some thermal issues; indeed, light source provides heating. The decision whether to use constant irradiation of pulsed light irradiation depends on the exact application and on the total desired irradiation. When the light exposure depends on the duration of a pulsed light, the net light time may be determined by the sum of the duration of each pulse.

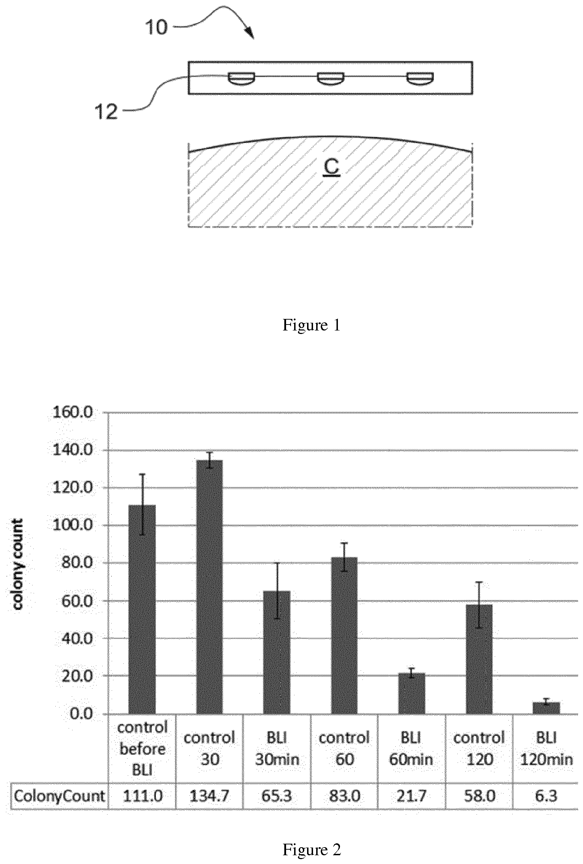

The light emitting element 12 used in the method of the invention is a device able to perform photobiomodulation. An example of such a light emitting element 12 is a light-emitting diode (LED or OLED, preferably LED), a LASER, or a lamp (such as filament lamp, gaz lamp) which is able to emit light at wavelengths within the ranges of 440 to 490 nm and having preferably a dominant emission wavelength comprised between 450-460 nm, as well as at a dominant emission wavelength of 450 or 453 nm. In figure 1, the light emitting element 12 comprises three light-emitting diodes. Alternatively, the light emitting element 12 may comprise one or more light-emitting diode (or lamp) able to emit a blue light having a wavelength ranging from 440 to 490 nm, having preferably a dominant emission wavelength comprised between 450 to 460 nm or having a dominant emission wavelength of about 450 or 453 nm.

For supplying electricity to the light emitting element 12, the light source device 10 may comprise a power source connected to the light emitting element 12. The power source may comprise an electric cable to connect to a power grid or a battery scavenger. Alternatively, the power source may be a battery, or a solar cell, preferably a battery. The light source device 10 is compact and able to communicate with a smartphone or a tablet thanks to a wireless communication protocol (Bluetooth or Bluetooth smart or Bluetooth Low Energy, NFC, Wifi, Lifi, Lora, Zigbee, preferably Bluetooth Low Energy).

For controlling the light emitting element 12, the light source device 10 may comprise at least one among a LED Driver, a sensor, a microchip processor, a control unit, a communication unit and an external port, an antenna, a memory.

A sensor may allow the light source device 10 to measure parameters of the contaminating and/or pathogenic agent. These parameters may be for example the temperature and the oxygenation level of the treated surface.

The microchip processor or the control unit may allow the light source device 10 to monitor the supply of electricity to the light emitting element 12 to guarantee an optimum or desired light exposure. For example, the microchip processor or the control unit may control whether the light exposure is continuous or in pulses as well as the frequency and the duration of the pulses depending on predetermined parameters or live parameters such as values measured by a sensor of the light source device 10.

Furthermore, a communication unit may allow a user to recover data from or transmit data to the light source device 10. For example, data may be transmitted to a smartphone or any other external device, notably an external device comprising a screen to display information useful to the user. The communication unit may be configured for wireless transmission or wired communication. In the case of a wired communication, the light source device 10 may comprise an external port connected to the communication unit for data transmission. Alternatively, the communication unit may be configured for both wireless and wired communication.

Moreover, the light source device 10 used in the method of the present invention may be included in a light source assembly (not shown) which comprises a product adapted to be in contact with a surface to be treated.For improving light effect, the light source assembly may be adapted to dispose the light emitting element 12 in a position wherein the light emitting element 12 is facing the support. In other words, the light source assembly is also adapted to place the light emitting element on the facing page of the support.

Furthermore, the light source device 10 used in the method of the present invention may irradiate light to the contaminating and/or pathogenic agent or to the support or medium through the product. In doing so, the light source device 10 can irradiate to the contaminating and/or pathogenic agent or to the support or medium, preferably the support without direct contact.

The method of the invention allows setting or predetermining of the distance between the light emitting element 12 and the support. Indeed, light intensity decreases with the square of the distance from the source of the light. For example, light 1 meter away from a source is four times as intense as light 2 meters from the same source. Therefore, setting the distance between the light emitting element 12 and the support allows monitoring the irradiance and thus the fluence provided to the said surface. The distance between the light emitting element 12 and the support may be predetermined from 0 to 50 mm, and preferably 0 to 20 mm in the case of a wound dressing for example. The distance between the light emitting element 12 and the support may be of several centimeters in the case of a lamp used alone for example.

For setting or predetermining the distance between the light emitting element 12 and the support, the dimension of the product may be chosen to predetermine or set the distance between the light emitting element 12 and the support or medium. Alternatively or in combination, the light source assembly may further comprise an adjustable element for adjusting the distance between the light emitting element 12 and the support.

The light source device 10 used in the present method may also be configured so that the light emitting element 12 may be selectively orientated to better target the contaminating and/or pathogenic agent to be irradiated. This orientation or homogenization of the light emitting element 12 allows the irradiation to be more adapted to the geometry and the characteristics of any contaminating and/or pathogenic agent or to the treated surface, support or medium. These advantages become even more significant when the light source device 10 comprises a plurality of light emitting elements 12. In this case, the light emitting elements 12 may be orientated independently from each other to widen the irradiated area.

Furthermore, the light source device 10 may comprise a lens for focusing the light onto the target the support or medium to make the irradiation more precise.

The product may be one among a dressing, a strip, a compression means, a Band-Aid, a patch, a gel and a rigid or flexible support, a film-forming composition or similar. Furthermore, in an embodiment of the light source assembly, the product may be arranged so that the light emitting element 12 is disposed on the interior of the product or in its inferior or superior surface.

The light source assembly used in the method of the present invention may be of any size or shape. The assembly may be 8×8 cm (or more 20x20 cm for instance) in size. The assembly may be 4×4 cm in size. The product may comprise an interior layer comprising a mesh material and a tissue gel. The mesh material allows exudate from a wound to which the dressing is applied to be absorbed into the dressing whilst allowing the tissue gel to flow through it so that it can be absorbed by a wound being treated.

For allowing the light source assembly to be reusable while avoiding repetitive cleanup, the product may be disposable and interchangeable. In other words, the product may be configured to be separated from the light source device 10 so that a same light source device 10 can be used several times without the need of a cleanup. It also allows changing the electronic elements included in the light source device 10 for maintenance, for example for recharging the battery.

Contaminating and/or pathogenic agent or the support or medium are irradiated with a light at wavelengths comprised between 440 to 440 nm, and preferably having a dominant emission wavelength comprised between 450 and 460 nm. More particularly, the chosen dominant emission wavelength may be of 450 or 453 nm. The method is performed *in vitro.* Bacteria, and more generally contaminating and/or pathogenic agent may be in culture.

To reduce their growth and number, contaminating and/or pathogenic agents may be irradiated to receive an effective fluence greater than 11 J/cm², preferably greater than 40 J/cm² and more preferably greater than 80 J/cm².

To reduce the growth and number of contaminating and/or pathogenic agents, light emitting source used in this method is greater than 20 mW/cm² and preferably comprised between 23 and 46 mW/cm².

More generally, the irradiation of light performed in this method may be set using all the different values of fluence, power intensity and time described above for the light source device 10 and the light source assembly.

The present method allows to obtain the unexpected technical effect of light consisting in at least inhibiting growth and reducing number of contaminating and/or pathogenic agent.

In particular, the method according to the invention is very useful for reducing growth and number of contaminating and/or pathogenic agents, for the treatment of liquid medium such as used waters, for surface decontamination, preferably through a photobiomodulation means. More specifically, the method according to the invention is very useful for reducing growth and number of contaminating and/or pathogenic agents.

The invention will be illustrated further by the following examples:

### Example 1: Effect of the blue light on the growth and number reduction of S. aureus

### Bacteria cells in suspension

1 mL of a *S*. *aureus* (ATCC 6538) solution at a concentration of 1.5 to 5×10⁷ CFU/ml were inoculated in Petri dishes comprising 9 mL of a mixture of 50% of buffered peptone water (0.1%) and 50% foetal veal serum (Simulated Wound Fluid or SWF), bacteria concentration was 1.5×10⁶ CFU/mL in the Petri dish.

Then, the Petri dishes inoculated with the bacteria are treated by a blue light.

### Light treatment

For the light treatment, OSRAM GD PSLR31.13 is used, with dominant emission wavelength of 450 nm (blue light). Dressings were directly irradiated with a power density of 23 or 46 mW/cm².

### Bacterial enumeration

Bacterial enumeration was conducted before light treatment, and after exposure to light to observe the reductive effect of light treatment on bacterial growth and number.

### Results

**Table 1: bacterial growth and number reduction observed after irradiation of S.aureus cells in suspension in SWF**

| Power density (mW/cm²) | Effective Fluence (J/cm²) | Time of exposure (min) | Ratio⁽¹⁾ | Bacterial reduction (Log) | Bacterial reduction (%) |
|---|---|---|---|---|---|
| 23 | 166 | 120 | 7.21 | 0,26 | 45 |
| 23 | 248 | 180 | 10.78 | 0,74 | 81 |
| 23 | 331 | 240 | 14.390 | 1,15 | Superior to 90 |
| 23 | 414 | 300 | 18 | 1,5 | Superior to 90 |
| 46 | 331 | 120 | 7.21 | 5,58 | Superior to 99 |

| | | | | | |
|---|---|---|---|---|---|
| ⁽¹⁾ Ratio between the effective fluence and the power density | | | | | |

The results show that the exposure of *S*. *aureus* to blue light (450 nm) with energy densities of greater than 41 J/cm², significantly inhibits bacterial growth and number and proliferation which well shows that such irradiation with blue light can be used to inhibit bacterial development on solid supports, and in particular on wounds and injuries.

### Example 2: Effect of the blue light on the growth and number reduction of P. aeruginosa

### Bacteria cells immobilized in wound dressings

*P. aeruginosa* (ATCC 15442) at a concentration of 1.5 to 5×10⁷ CFU/ml were inoculated on the surface of pre-wetted dressings. The concentration of bacteria was about 5×10⁶ CFU/dressing.

Then, the dressings inoculated with the bacteria are treated by a blue light.

### Light treatment

For the light treatment, OSRAM GD PSLR31.13 is used, with dominant emission wavelength of 450 nm (blue light). Dressings were directly irradiated with a power density of 23 or 46 mW/cm².

### Bacterial enumeration

Bacterial enumeration was conducted before light treatment, and after exposure to light to observe the reductive effect of light treatment on bacterial growth and number.

### Results

**Table 2: bacterial number and growth reduction observed after irradiation of inoculated dressings with P.aeruginosa**

| power density (mW/cm ²) | Effective Fluence (J/cm²) | Time of exposure (mins) | Ratio⁽¹⁾ | Bacterial reduction (Log) | Bacterial reduction (%) |
|---|---|---|---|---|---|
| 23 | 10 | 7,5 | 0.43 | 0.02 | 4 |
| 23 | 41 | 30 | 1.78 | 0,14 | 27 |
| 23 | 83 | 60 | 3.60 | 0,39 | 59 |
| 23 | 166 | 120 | 7.21 | 1,04 | Superior to 90 |
| 23 | 414 | 300 | 18 | 4,56 | Superior to 99 |
| 46 | 166 | 60 | 3.60 | 3,53 | Superior to 99 |
| 46 | 414 | 150 | 9 | 4,74 | Superior to 99 |

| | | | | | |
|---|---|---|---|---|---|
| ⁽¹⁾ Ratio between the effective fluence and the power density | | | | | |

The results show that the exposure of *P. aeruginosa* to blue light (450 nm) with energy densities greater than 41 J/cm², significantly reduces bacterial growth and number which well shows that such irradiation with blue light can be used to inhibit bacterial development on solid supports, and in particular on wounds and injuries.

In conclusion, the results exhibit that the exposure of bacteria (whatever the considered specie, Gram-positive or Gram-negative) to blue light (specifically at 450 nm or 453 nm) with energy densities greater than 11 J/cm², preferably greater than 40 J/cm² (more precisely, 41 J/cm²), and irradiance values greater than 20 mW/cm², significantly reduces the bacterial growth and number.

### Example 3: Effect of the blue light on the growth and number of E. coli

### Bacterial culture

0.5 mL of an *Escherichia coli* (strain K12) (Taxon identifier: 83333) solution at a concentration of 1×10⁶ CFU/ml in NaCl 0.9% were inoculated in 4.5 mL nutrient broth (8 g/L (Merck)). Afterwards, a dilution series is prepared and then after irradiation by a blue light, bacteria were seeded on plates and number of colonies was counted 24 hours later.

### Light treatment

For the light treatment, Lumileds Luxeon Rebel LXML-PR01-0275 from Koninklijke Philips N.V. (Eindhoven/Netherlands) was used, with a dominant emission wavelength of 453 nm (blue light).

Suspensions were directly irradiated with a power density of 10 or 23 mW/cm².

### Bacterial enumeration

Bacterial enumeration was conducted before light treatment, and after incubation to observe the inhibitory effect of light treatment on bacterial growth and number.

### Results

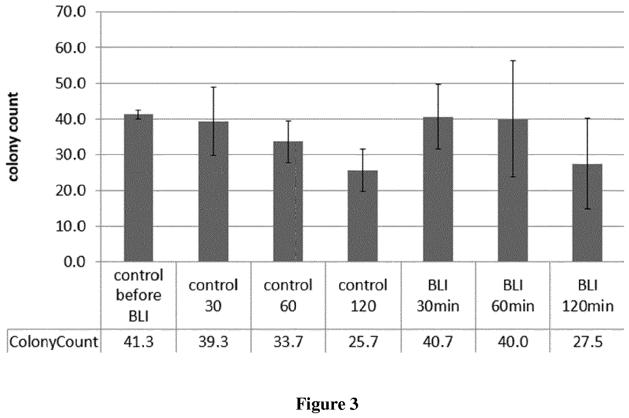

Results are shown in figure 2 and 3.

### Nb:

- BLI means Blue light irradiation
- Control doesn't receive any light irradiation whatever the exposure time considered
- Numbers following BLI or control mention, express time (with exposure to blue light (BLI) or without exposure (control)) after which colony count has been made.

**Table 3: Correspondence between time of exposure, power density used and fluence definition for each condition**

| power density (mW/cm²) | Time of exposure | Effective Fluence (J/cm²) |
|---|---|---|
| 23 | 30 min | 41 |
| 23 | 60 min | 83 |
| 23 | 120 min | 166 |
| 10 | 30 min | 18 |
| 10 | 60 min | 36 |
| 10 | 120 min | 72 |

Figure 2 represents a histogram comparing the effect on colony count of E. *Coli* in blue light irradiation conditions (453 nm, defined as BLI) Vs without irradiation (defined as control), and for irradicance values of 23 mW/cm².

Figure 3 represents a histogram comparing the effect on colony count of E. *Coli* in blue light irradiation conditions (453 nm, defined as BLI) Vs without irradiation (defined as control), and for irradicance values of 10 mW/cm².

The results show that the exposure of *E. coli* to blue light (453 nm) with a device having the irradiance feature of 10 and 23 mW/cm² induce respective different issues too. More precisely, blue light irradiation of *E. Coli* at 23 mW/cm² (fig. 2) induces a drastic and linear reduction of the number of colony counted whatever the time of exposure. On the contrary, there is no significant effect on the colony number measured between *E. Coli* treated with blue light (10 mW/cm²) and control group for the same time of exposure (fig. 3).

Indeed, examples 1 to 3 show that a device comprising a light emitting element for emitting a light having the following characteristics:
a wavelength ranging from 435 to 520 nm, and
a power density greater than 20 mW/cm²,
the light source device provides an effective fluence to any contaminating and/or pathogenic agent greater than 11 J/cm² exhibit a specific and surprising effect on the growth and number reduction of contaminating and/or pathogenic agent, preferably bacteria, whatever the specie (Gram-positive or Gram-negative) of bacteria.

## Claims

1. An *in vitro* method for inhibiting growth and reducing number of contaminating and/or pathogenic agents, the method comprising the step of exposing the contaminating and/or pathogenic agents to a light source device (10) comprising a light emitting element (12) emitting a blue light having a wavelength ranging from 440 to 490 nm, and
the light source device (10) providing an effective fluence to the contaminating and/or pathogenic agents greater than 11 J/cm², and a power density greater than 20 mW/cm²,
wherein the ratio between the effective fluence and the power density is greater than 1.7 J/ mW preferably greater than 3 J/ mW.

2. The method of claim 1, wherein the dominant emission wavelength ranges from 450 to 460 nm.

3. The method according to claims 1 or 2, wherein the light source device (10) provides an effective fluence to the contaminating and/or pathogenic agent greater than 40 J/cm², preferably greater than 80 J/cm².

4. The method according to any one of claims 1 to 3, wherein the light emitting element (12) provides a power density ranging from 23 to 46 mW/cm².

5. The method according to any one of claims 1 to 4, wherein said light emitting element (12) comprises at least one LED.

6. The method according to any one of claims 1 to 5, further comprising a power source providing electrical power to said light emitting element (12).

7. The method according to any one of claim 1 to 6, wherein the contaminating and/or pathogenic agent is a microorganism such as bacteria, yeasts or fungi, preferably bacteria.

8. The method according to any one of claims 1 to 7, wherein the contaminating and/or pathogenic agent is a bacteria, in particular a Gram-positive or Gram-negative bacteria, preferably chosen from *S*. *aureus* and *P. aeruginosa.*

9. The method according to any one of claims 1 to 6, for the treatment of fluid medium or surface decontamination, preferably through a photobiomodulation means.

10. The method according to any one of claims 1 to 6, for the disinfection of wounds, mucosa, and skin, preferably through a photobiomodulation means.

## Patentansprüche

1. *In-vitro*-Verfahren zum Hemmen eines Wachstums und Reduzieren einer Anzahl kontaminierender und/oder pathogener Agenzien, wobei das Verfahren den Schritt eines Aussetzens der kontaminierenden und/oder pathogenen Agenzien einer Lichtquellenvorrichtung (10) umfasst, welche ein lichtemittierendes Element (12) umfasst, welches ein blaues Licht emittiert, das eine Wellenlänge aufweist, welche im Bereich von 440 bis 490 nm liegt, und
wobei die Lichtquellenvorrichtung (10) den kontaminierenden und/oder pathogenen Agenzien eine wirksame Fluenz von mehr als 11 J/cm² und eine Energiedichte von mehr als 20 mW/cm² bereitstellt,
wobei das Verhältnis zwischen der wirksamen Fluenz und der Energiedichte größer als 1,7 J/mW, vorzugsweise größer als 3 J/mW, ist.

2. Verfahren nach Anspruch 1, wobei die dominante Emissionswellenlänge im Bereich von 450 bis 460 nm liegt.

3. Verfahren nach den Ansprüchen 1 oder 2, wobei die Lichtquellenvorrichtung (10) eine wirksame Fluenz für das kontaminierende und/oder pathogene Agens von mehr als 40 J/cm², vorzugsweise mehr als 80 J/cm², bereitstellt.

4. Verfahren nach einem der Ansprüche 1 bis 3, wobei das lichtemittierende Element (12) eine Energiedichte bereitstellt, welche im Bereich von 23 bis 46 mW/cm² liegt.

5. Verfahren nach einem der Ansprüche 1 bis 4, wobei das lichtemittierende Element (12) wenigstens eine LED umfasst.

6. Verfahren nach einem der Ansprüche 1 bis 5, ferner umfassend eine Energiequelle, welche dem lichtemittierenden Element (12) elektrische Energie bereitstellt.

7. Verfahren nach einem der Ansprüche 1 bis 6, wobei das kontaminierende und/oder pathogene Agens ein Mikroorganismus, wie etwa Bakterien, Hefen oder Pilze, vorzugsweise Bakterien, ist.

8. Verfahren nach einem der Ansprüche 1 bis 7, wobei das kontaminierende und/oder pathogene Agens ein Bakterium, insbesondere ein grampositives oder gramnegatives Bakterium, ist, vorzugsweise ausgewählt aus *S*. *aureus* und *P. aeruginosa.*

9. Verfahren nach einem der Ansprüche 1 bis 6, zur Behandlung eines Fluidmediums oder zur Oberflächendekontamination, vorzugsweise durch ein Photobiomodulationsmittel.

10. Verfahren nach einem der Ansprüche 1 bis 6, zur Desinfektion von Wunden, Schleimhäuten und Haut, vorzugsweise durch ein Photobiomodulationsmittel.

## Revendications

1. Procédé *in vitro* d'inhibition de la croissance et de réduction du nombre d'agents contaminants et/ou pathogènes, le procédé comprenant l'étape d'exposition des agents contaminants et/ou pathogènes à un dispositif source de lumière (10) comprenant un élément émetteur de lumière (12) émettant une lumière bleue d'une longueur d'onde comprise entre 440 et 490 nm, et
le dispositif source de lumière (10) fournissant une fluence effective aux agents contaminants et/ou pathogènes supérieure à 11 J/cm², et une densité de puissance supérieure à 20 mW/cm²,
le rapport entre la fluence effective et la densité de puissance étant supérieur à 1,7 J/mW de préférence supérieur à 3 J/mW.

2. Procédé selon la revendication 1, la longueur d'onde d'émission dominante allant de 450 à 460 nm.

3. Procédé selon les revendications 1 ou 2, le dispositif source de lumière (10) fournissant une fluence effective à l'agent contaminant et/ou pathogène supérieure à 40 J/cm², de préférence supérieure à 80 J/cm².

4. Procédé selon l'une quelconque des revendications 1 à 3, l'élément émetteur de lumière (12) fournissant une densité de puissance allant de 23 à 46 mW/cm².

5. Procédé selon l'une quelconque des revendications 1 à 4, ledit élément émetteur de lumière (12) comprenant au moins une DEL.

6. Procédé selon l'une quelconque des revendications 1 à 5, comprenant en outre une source d'alimentation fournissant une puissance électrique audit élément émetteur de lumière (12).

7. Procédé selon l'une quelconque des revendications 1 à 6, l'agent contaminant et/ou pathogène étant un micro-organisme tel que des bactéries, des levures ou des champignons, de préférence des bactéries.

8. Procédé selon l'une quelconque des revendications 1 à 7, l'agent contaminant et/ou pathogène étant une bactérie, en particulier une bactérie à Gram positif ou à Gram négatif, de préférence choisie parmi *S. aureus* et *P. aeruginosa.*

9. Procédé selon l'une quelconque des revendications 1 à 6, pour le traitement d'un milieu fluide ou de décontamination de surface, de préférence par un moyen de photobiomodulation.

10. Procédé selon l'une quelconque des revendications 1 à 6, pour la désinfection des plaies, des muqueuses et de la peau, de préférence par un moyen de photobiomodulation.
